(19) Europäisches Patentamt
European Patent Office
Office européen des brevets

(11) Veröffentlichungsnummer: **0 151 312**
**B1**

(12) # EUROPÄISCHE PATENTSCHRIFT

(45) Veröffentlichungstag der Patentschrift: **06.04.88**

(51) Int. Cl.⁴: **C 07 C 161/00** // C07D285/00

(21) Anmeldenummer: **84116311.6**

(22) Anmeldetag: **27.12.84**

(54) **N'-Acylhydrazin-N-thiocarbonsäure-O-carbamoylmethylester.**

(30) Priorität: **04.01.84 DE 3400169**

(43) Veröffentlichungstag der Anmeldung: **14.08.85 Patentblatt 85/33**

(45) Bekanntmachung des Hinweises auf die Patenterteilung: **06.04.88 Patentblatt 88/14**

(84) Benannte Vertragsstaaten: **BE CH DE FR GB IT LI NL**

(56) Entgegenhaltungen: **EP-A-0 148 501**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**

**EP 0 151 312 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue N'-Acylhydrazin-N-thiocarbonsäure-O-carbamoylmethylester, mehrere Verfahren zu ihrer Herstellung sowie ihre Verwendung als Zwischenprodukte zur Synthese von 1,3,4-Thiadiazolyloxyacetamiden, welche herbizide Eigenschaften besitzen.

Es ist bereits bekannt, daß man herbizide Heteroaryloxyacetamide erhält, wenn man 2-Halogenheteroaromaten mit Hydroxyacetamiden umsetzt (vgl. z. B. DE-OS-29 14 003 und DE-OS-30 04 326 [=EP 18 497]; DE-OS-29 46 526 [=EP 29 171] und DE-OS-32 18 482).

Besonders im Fall der 1,3,4-Thiadiazol-2-yloxyacetamide erhält man jedoch bei Verwendung von 2-Halogen-1,3,4-thiadiazolen als Ausgangsstoffe die gewünschten herbiziden Endprodukte nur in nicht zufriedenstellender Ausbeute und Reinheit.

Es wurden neue N'-Acylhydrazin-N-thiocarbonsäure-O-carbamoylmethylester der allgemeinen Formel (I),

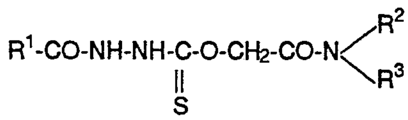

(I)

in welcher

$R_1$ für Alkyl, Alkoxy, Alkylthio, Halogenalkyl, Aralkyl, Aralkoxy, Aralkylthio oder gegebenenfalls substituiertes Aryl steht, und

$R_2$ und $R_3$ unabhängig voneinander für Wasserstoff, Alkyl, Alkenyl, Alkinyl, für jeweils gegebenenfalls substituiertes Cycloalkyl oder Cycloalkenyl, für Halogenalkyl, Alkoxyalkyl und Alkoxy, für Aralkyl und gegebenenfalls substituiertes Aryl stehen, oder

$R_2$ und $R_3$ zusammen mit dem Stickstoffatom, an welches sie gebunden sind, für einen gegebenenfalls substituierten, gesättigten oder ungesättigten Heterocyclus stehen, der weitere Heteroatome enthalten kann,

gefunden.

Weiterhin wurde gefunden, daß man die neuen N'-Acylhydrazin-N-thiocarbonsäure-O-carbamoylmethylester der allgemeinen Formel (I),

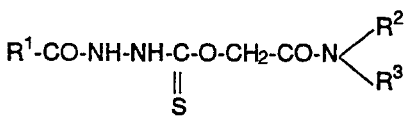

(I)

in welcher

$R_1$ für Alkyl, Alkoxy, Alkylthio, Halogenalkyl, Aralkyl, Aralkoxy, Aralkylthio oder für gegebenenfalls substituiertes Aryl steht und

$R_2$ und $R_3$ unabhängig voneinander für Wasserstoff, Alkyl, Alkenyl, Alkinyl, für gegebenenfalls substituiertes Cycloalkyl oder Cycloalkenyl, für Halogenalkyl, Alkoxyalkyl, Alkoxy, für Aralkyl oder für gegebenenfalls substituiertes Aryl stehen oder

$R_2$ und $R_3$ gemeinsam mit dem Stickstoffatom, an welches sie gebunden sind, für einen gegebenenfalls substituierten, gesättigten oder ungesättigten Heterocyclus stehen, der weitere Heteroatome enthalten kann,

erhält, wenn man

(a) Hydrazin-N-thiocarbonsäure-O-carbamoylmethylester der allgemeinen Formel (II),

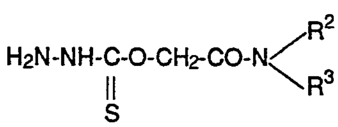

(II)

in welcher

$R_2$ und $R_3$ die oben angegebenen Bedeutungen haben, mit Acylierungsmitteln der Formel (III),

$$R^1\text{-CO-X} \qquad \text{(III)}$$

in welcher

$R_1$ die oben angegebene Bedeutung hat und

X für Halogen oder Alkoxy steht,

gegebenenfalls in Gegenwart eines Verdünnungsmittels sowie gegebenenfalls in Gegenwart eines Säurebindemittels umsetzt, oder wenn man

(b) O-Carbamoylmethyl-S-carboxymethyl-dithiocarbonate der Formel (IV),

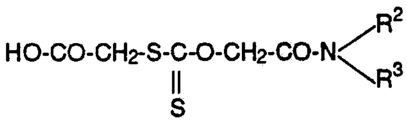 (IV)

in welcher
$R_2$ und $R_3$ die oben angegebenen Bedeutungen haben,
mit N-Acylhydrazinen der Formel (V),

$R^1$-CO-NH-$NH_2$ (V)

in welcher
$R_1$ die oben angegebene Bedeutung hat,
gegebenenfalls in Gegenwart eines Verdünnungsmittels sowie gegebenenfalls in Gegenwart eines Säurebindemittels umsetzt, oder wenn man
c) Hydroxyacetamide der Formel (VI)

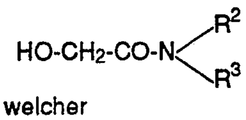 (VI)

in welcher
$R_2$ und $R_3$ die oben angegebenen Bedeutungen haben,
nacheinander in einem 'Eintopfverfahren' zunächst mit Schwefelkohlenstoff in Gegenwart einer Base, dann mit einem Chloressigsäure-Alkalisalz und zuletzt mit einem N-Acylhydrazin der Formel (V),

$R^1$-CO-NH-$NH_2$ (V)

in welcher
$R_1$ die oben angegebene Bedeutung hat,
gegebenenfalls in Gegenwart eines Verdünnungsmittels umsetzt.

Schließlich wurde gefunden, daß die neuen N'-Acylhydrazin-N-thiocarbonsäure-O-carbamoylmethylester der Formel (I) interessante Zwischenprodukte zur Herstellung von Pflanzenschutzmitteln sind, welche herbizide Eigenschaften besitzen.

Überraschenderweise erhält man bei Verwendung der neuen N'-Acylhydrazin-N-thiocarbonsäure-O-carbamoylmethylester der Formel (I) als Ausgangsstoffe herbizide 1,3,4-Thiadiazol-2-yloxyacetamide in wesentlich besseren Ausbeuten und höherer Reinheit als bei Verwendung der aus dem Stand der Technik bekannten 2-Halogen-1,3,4-thiadiazole als Ausgangsstoffe.

Die erfindungsgemäßen N'-Acylhydrazin-N-thiocarbonsäure-O-carbamoylmethylester sind durch die Formel (I) allgemein definiert.

Bevorzugt sind Verbindungen der Formel (I), bei welchen

$R^1$ für geradkettiges oder verzweigtes Alkyl, Alkoxy oder Alkylthio mit jeweils 1 bis 6 Kohlenstoffatomen, für Halogenalkyl mit 1 bis 6 Kohlenstoffatomen und 1 bis 13 Halogenatomen, (insbesondere Fluor, Chlor und Brom), für Aralkyl, Aralkoxy und Aralkylthio mit 6 bis 10 Kohlenstoffatomen im Arylteil und 1 bis 2 Kohlenstoffatomen im Alkylteil sowie für gegebenenfalls einfach oder mehrfach, gleich oder verschieden substituiertes Aryl mit 6 bis 10 Kohlenstoffatomen steht, wobei als Substituenten infrage kommen: Alkyl und Alkoxy mit jeweils 1 bis 4 Kohlenstoffatomen, Halogenalkyl mit 1 bis 4 Kohlenstoffatomen und 1 bis 5 Halogenatomen, insbesondere Fluor, Chlor oder Brom, und

$R^2$ und $R^3$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen, wobei als Substituenten insbesondere Alkylreste mit 1 bis 4 Kohlenstoffatomen infrage kommen, für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen, insbesondere Fluor, Chlor und Brom, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten infrage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor, Chlor oder Brom, sowie Nitro, oder

$R^2$ und $R^3$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff und

Sauerstoff enthalten kann, wobei als Substituenten infrage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

Besonders bevorzugt sind Verbindungen der Formel (I), bei welchen

$R^1$ für geradkettiges oder verzweigtes Alkyl, Alkoxy oder Alkylthio mit jeweils 1 bis 4 Kohlenstoffatomen, für Halogenalkyl mit 1 bis 4 Kohlenstoffatomen und 1 bis 9 gleichen oder verschiedenen Halogenatomen, insbesondere Fluor, Chlor oder Brom, für Benzyl oder Benzylthio sowie für gegebenenfalls einfach bis dreifach, gleich oder verschieden substituiertes Phenyl mit den besonders bevorzugten Substituenten: Methyl, Methoxy und Trifluormethyl, steht, und

$R^2$ und $R^3$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, gegebenenfalls ein- bis dreifach, gleich oder verschieden durch Methyl oder Ethyl substituiertes Cycloalkyl bzw. Cycloalkenyl mit 5 bis 7 Kohlenstoffatomen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach, gleich oder verschieden substituiertes Phenyl stehen, wobei als Substituenten besonders bevorzugte sind: Methyl, Ethyl, Methoxy, Methylthio, Trifluormethyl, Trifluormethoxy, Trifluormethylthio, Fluor, Chlor, Nitro, oder

$R^2$ und $R^3$ zusammen mit dem Stickstoffatom, an das sie gebunden sind, für einen der gegebenenfalls ein- bis dreifach, gleich oder verschieden substituierten Heterocyclen

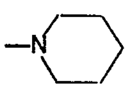 ; 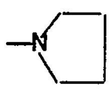 ; 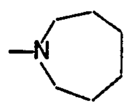 ; 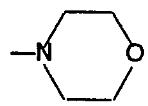 ; 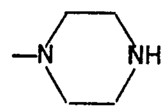 ;

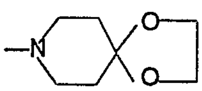 ; 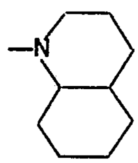 ; 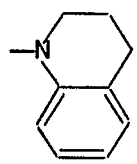 stehen,

wobei als Substituenten besonders bevorzugt sind: Methyl, Ethyl und Phenyl.

Im einzelnen seien außer den bei den Herstellungsbeispielen genannten Verbindungen die folgenden Verbindungen der allgemeinen Formel (I) genannt:

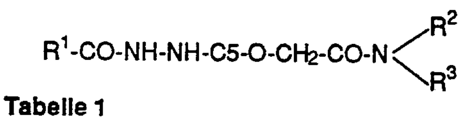 (I)

**Tabelle 1**

| $R^1$ | $R^2$ | $R^3$ |
|---|---|---|
| $F_3C$- | $C_2H_5$ | $C_2H_5$- |
| $F_3C$- | $C_2H_5$ | $C_2H_5$- |
| $F_3C$- | n-$C_3H_7$ | n-$C_3H_7$ |
| $F_3C$- | -$CH_2$-CH=$CH_2$ | -$CH_2$-CH=$CH_2$ |
| $F_3C$- | -$CH_2$-$CH_2$-$CH_2$-$CH_2$-$CH_2$ | |
| $F_3C$- | -$OCH_3$ | 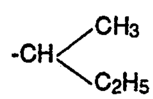 |
| $F_3C$- | 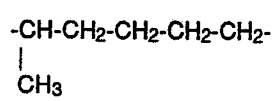 | |
| $F_3C$- | 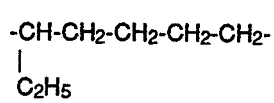 | |
| $F_2ClC$- | $CH_3$ | $C_6H_5$ |
| $F_2ClC$- | $C_2H_5$ | $C_2H_5$ |
| $F_2ClC$- | n-$C_3H_7$ | n-$C_3H_7$ |
| $F_2ClC$- | -$CH_2$-CH=$CH_2$; | -$CH_2$-CH=$CH_2$ |
| $F_2ClC$- | -$CH_2$-$CH_2$-$CH_2$-$CH_2$-$CH_2$- | |

**Fortsetzung Tabelle 1**

| $R^1$ | $R^2$ | $R^3$ |
|---|---|---|
| $F_2ClC-$ | $-CH-CH_2-CH_2-CH_2-CH_2-$<br>\|<br>$CH_3$ | |
| $F_2ClC-$ | $-CH-CH_2-CH_2-CH_2-CH_2-$<br>\|<br>$C_2H_5$ | |
| $Cl_2FC-$ | $CH_3$ | $C_6H_5$ |
| $Cl_2FC-$ | $C_2H_5$ | $C_6H_5$ |
| $Cl_2FC-$ | $n-C_3H_7$ | $n-C_3H_7$ |
| $Cl_2FC-$ | $CH_2=CH-CH_2-$ | $CH_2=CH-CH_2-$ |
| $Cl_2FC-$ | $C_2H_5$ | $C_2H_5$ |
| $Cl_2FC-$ | $-CH-CH_2-CH_2-CH_2-CH_2-$<br>\|<br>$CH_3$ | |
| $Cl_2FC-$ | $-CH_2-CH_2-CH_2-CH_2-CH_2-$ | |
| $Cl_2FC-$ | $-CH-CH_2-CH_2-CH_2-CH_2-$<br>\|<br>$C_2H_5$ | |
| $Cl_3C-$ | $CH_3$ | $C_6H_5-$ |
| $Cl_3C-$ | $C_2H_5$ | $C_6H_5-$ |
| $Cl_3C-$ | $C_2H_5$ | $C_2H_5-$ |
| $Cl_3C-$ | $CH_2=CH-CH_2-$ | $CH_2=CH-CH_2-$ |
| $Cl_3C-$ | $-CH_2-CH_2-CH_2-CH_2-CH_2-$ | |
| $Cl_3C-$ | $-CH-CH_2-CH_2-CH_2-CH_2-$<br>\|<br>$CH_3$ | |
| $Cl_3C-$ | $-CH-CH_2-CH_2-CH_2-CH_2-$<br>\|<br>$C_2H_5$ | |
| $CH_3-$ | $CH_3$ | $C_6H_5$ |
| $CH_3-$ | $C_2H_5$ | $C_6H_5$ |
| $CH_3-$ | $C_2H_5$ | $C_2H_5$ |
| $CH_3-$ | $n-C_3H_7$ | $n-C_3H_7$ |
| $CH_3-$ | $CH_2=CH-CH_2-$ | $CH_2=CH-CH_2-$ |
| $CH_3-$ | $-CH_2-CH_2-CH_2-CH_2-CH_2-$ | |
| $CH_3-$ | $-CH_2-CH_2-O-CH_2-CH_2-$ | |
| $CH_3-$ | $-CH-CH_2-CH_2-CH_2-CH_2-$<br>\|<br>$CH_3$ | |
| $CH_3-$ | $-CH-CH_2-CH_2-CH_2-CH_2-$<br>\|<br>$C_2H_5$ | |
| $C_2H_5$ | $CH_3$ | $C_6H_5$ |
| $C_2H_5$ | $C_2H_5$ | $C_6H_5$ |
| $C_2H_5$ | $C_2H_5$ | $C_2H_5$ |
| $C_2H_5$ | $n-C_3H_7$ | $n-C_3H_7$ |
| $CH_3-(CH_2)_2-$ | $CH_3$ | $C_6H_5$ |
| $CH_3-(CH_2)_2-$ | $n-C_3H_7$ | $n-C_3H_7$ |

**Fortsetzung Tabelle 1**

| $R^1$ | $R^2$ | $R^3$ |
|---|---|---|
| $CH_3-(CH_2)_2-$ | $CH_2=CH-CH_2-$ | $CH_2=CH-CH_2-$ |
| $CH_3-(CH_2)_2-$ | $C_2H_5$ | $C_2H_5$ |
| $CH_3-(CH_2)_2-$ | $C_2H_5$ | $C_6H_5$ |
| $CH_3-(CH_2)_2-$ | $-CH_2-CH_2-CH_2-CH_2-CH_2-$ | |
| $CH_3-(CH_2)_2-$ | $-CH-CH_2-CH_2-CH_2-CH_2-$<br>\|<br>$C_2H_5$ | |
| $(CH_3)_2CH-$ | $CH_3$ | $C_6H_5$ |
| $(CH_3)_2CH-$ | $C_2H_5$ | $C_6H_5$ |
| $(CH_3)_2CH-$ | $C_2H_5$ | $C_2H_5$ |
| $(CH_3)_2CH-$ | $n-C_3H_7$ | $n-C_3H_7$ |
| $(CH_3)_2CH-$ | $CH_2=CH-CH_2-$ | $CH_2=CH-CH_2-$ |
| $(CH_3)_2CH-$ | $HC\equiv C-CH_2-$ | $HC\equiv C-CH_2-$ |
| $(CH_3)_2CH-$ | $-CH_2-CH_2-CH_2-CH_2-CH_2-$ | |
| $(CH_3)_2CH-$ | $-CH-CH_2-CH_2-CH_2-CH_2-$<br>\|<br>$CH_3$ | |
| $(CH_3)_2CH-$ | $-CH-CH_2-CH_2-CH_2-CH_2-$<br>\|<br>$C_2H_5$ | |
| $ClH_2C-$ | $CH_3$ | $C_6H_5$ |
| $ClH_2C-$ | $CH_2=CH-CH_2-$ | $CH_2=CH-CH_2-$ |
| $ClH_2C-$ | $n-C_3H_7$ | $n-C_3H_7$ |
| $ClH_2C-$ | $-CH-CH_2-CH_2-CH_2-CH_2-$<br>\|<br>$C_2H_5$ | |
| $Cl_2HC$ | $CH_3$ | $C_6H_5$ |
| $Cl_2HC-$ | $C_2H_5$ | $C_2H_5$ |
| $Cl_2HC-$ | $n-C_3H_7$ | $n-C_3H_7$ |
| $Cl_2HC-$ | $CH_2=CH-CH_2-$ | $CH_2=CH-CH_2-$ |
| $F_2HC-$ | $-CH_2-CH_2-CH_2-CH_2-CH_2-$ | |
| $F_2HC-$ | $CH_3$ | $C_6H_5$ |
| $F_2HC-$ | $-CH-CH_2-CH_2-CH_2-CH_2-$<br>\|<br>$C_2H_5$ | |
| $F_2HC-$ | $n-C_3H_7$ | $n-C_3H_7$ |
| $FH_2C-$ | $CH_2=CH-CH_2-$ | $CH_2=CH-CH_2-$ |
| $FH_2C-$ | $CH_3$ | $C_6H_5$ |
| $FH_2C-$ | $C_2H_5$ | $C_6H_5$ |
| $FH_2C-$ | $n-C_3H_7$ | $n-C_3H_7$ |
| $F_3C-CF_2-CF_2-$ | $CH_3$ | $C_6H_5$ |
| $F_3C-CF_2-CF_2-$ | $n-C_3H_7$ | $n-C_3H_7$ |
| $F_3C-CF_2-CF_2-$ | $CH_2=CH-CH_2-$ | $CH_2=CH-CH_2-$ |
| $F_3C-CF_2-CF_2-$ | $-CH-CH_2-CH_2-CH_2-CH_2-$<br>\|<br>$C_2H_5$ | |
| $FClHC-$ | $CH_3$ | $C_6H_5$ |
| $FClHC-$ | $n-C_3H_7$ | $n-C_3H_7$ |
| $FClHC-$ | $CH_2=CH-CH_2-$ | $CH_2=CH-CH_2-$ |

**Fortsetzung Tabelle 1**

| $R^1$ | $R^2$ | $R^3$ |
|---|---|---|
| FClHC- | $-CH_2-CH_2-CH_2-CH_2-CH_2-$ | |
| FClHC- | $-\underset{CH_3}{\underset{\vert}{CH}}-CH_2-CH_2-CH_2-CH_2-$ | |
| $C_6H_5$ | $CH_3$ | $C_6H_5$ |
| $C_6H_5$ | $C_2H_5$ | $C_6H_5$ |
| $C_6H_5$ | $n-C_3H_7$ | $n-C_3H_7$ |
| $C_6H_5$ | $CH_2=CH-CH_2-$ | $CH_2=CH-CH_2-$ |
| $C_6H_5$ | $-CH_2-CH_2-CH_2-CH_2-CH_2-$ | |

Verwendet man beispielsweise Hydrazinthiocarbonsäure-O-(N-methyl-N-phenylcarbamoylmethyl)-ester und Trifluoressigsäureethylester, so läßt sich der Reaktionsablauf des erfindungsgemäßen Verfahrens (a) durch das folgende Formelschema darstellen:

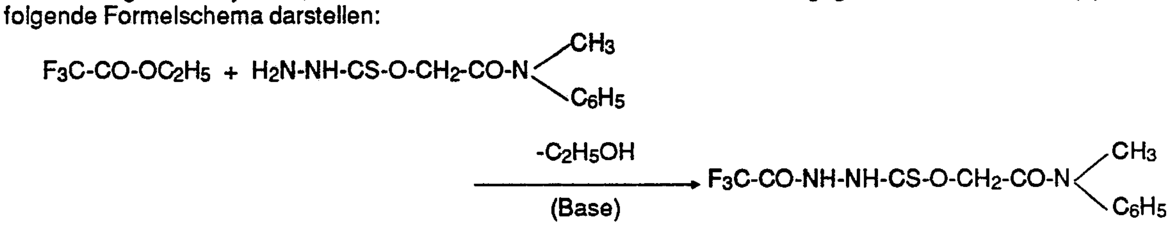


Verwendet man beispielsweise S-Carboxymethyl-O-(N-methylN-phenylcarbamoylmethyl)-dithiocarbonat und N-Acetylhydrazin als Ausgangsstoffe, so läßt sich der Reaktionsablauf des erfindungsgemäßen Verfahrens (b) durch das folgende Formelschema darstellen:

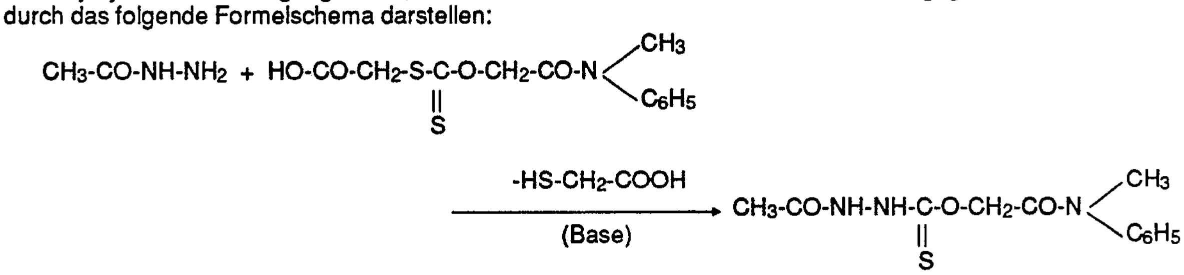


Verwendet man beispielsweise Glykolsäurepiperidid, Schwefelkohlenstoff, Kaliumhydroxid, Chloressigsäure-Natriumsalz und N-Acetylhydrazin als Ausgangsstoffe, so läßt sich der Reaktionsablauf des erfindungsgemäßen Verfahrens (c) durch das folgende Formelschema darstellen:

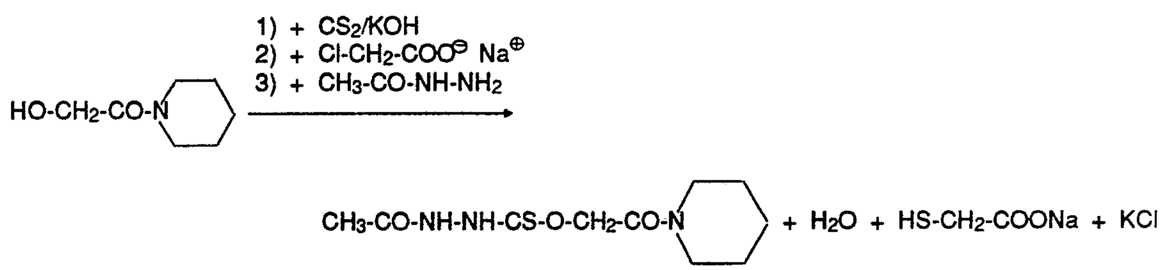


Die als Ausgangsstoffe für das erfindungsgemäße Verfahren (a) benötigten Hydrazin-thiocarbonsäure-O-carbamoylmethylester sind durch die Formel (II) allgemein definiert. In dieser Formel (II) stehen $R^2$ und $R^3$ vorzugsweise für diejenigen Reste, die schon bei der Beschreibung der erfindungsgemäßen Stoffe der Formel (I) als bevorzugt für diese Substituenten genannt wurden.

Die Hydrazin-thiocarbonsäure-O-carbamoylmethylester sind noch nicht bekannt.

Man erhält sie entweder, wenn man O-Carbamoylmethyl-S-carboxymethyl-dithiocarbonate der Formel (IV),

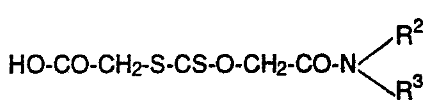 (IV)

in welcher

$R^2$ und $R^3$ die oben angegebenen Bedeutungen haben,

mit Hydrazinhydrat gegebenfalls in Gegenwart eines Verdünnungsmittels, wie beispielsweise Wasser, und gegebenenfalls in Gegenwart eines Säurebindemittels, wie beispielsweise Natriumhydrogencarbonat, bei Temperaturen zwischen -20°C und +50°C umsetzt, oder wenn man Hydroxyacetamide der Formel (VI),

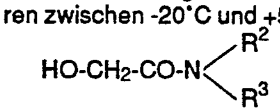
(VI)

in welcher
$R^2$ und $R^3$ die oben angegebenen Bedeutungen haben,
nacheinander in einem 'Eintopfverfahren' zunächts mit Schwefelkohlenstoff in Gegenwart einer Barse, wie z. B. Alkalimetallhydroxid, dann mit einem Chloressigsäure-Alkalisalz, wie beispielsweise Chloressigsäurenatriumsalz, und zuletzt mit Hydrazinhydrat gegebenenfalls in Gegenwart eines Verdünnungsmittels, wie beispielsweise Wasser, bei Temperaturen zwischen 0°C und +60°C umsetzt.
Die weiterhin zur Durchführung des erfindungsgemäßen Verfahrens (a) als Ausgangsstoffe benötigten Acylierungsmittel sind durch die Formel (III) allgemaine definiert. In dieser Formel (III) steht $R^1$ vorzugsweise für diejenigen Reste, die schon im Zusammenhang mit der Beschreibung der erfindungsgemäßen Stoffe der Formel (I) als bevorzugt für diesen Substituenten genannt wurden. X steht vorzugsweise für Chlor, Brom, Methoxy oder Ethoxy. Die Acylierungsmittel der Formel (III) sind allgemein bekannte Verbindungen der organischen Chemie.

Die zur Durchführung des erfindungsgemäßen Verfahrens (b) und zur Herstellung der Vorprodukte der Formel (II) als Ausgangsstoffe benötigten O-Carbamoylmethyl-S-carboxymethyl-dithiocarbonate sind durch die Formel (IV) allgemein definiert. In dieser Formel (IV) stehen $R^2$ und $R^3$ vorzugsweise für diejenigen Substituenten, die schon bei der Beschreibung der erfindungsgemäßen Stoffe der Formel (I) als bevorzugt für diese Reste genannt wurden.

Die O-Carbamoylmethyl-S-carboxymethyl-dithiocarbonate der Formel (IV) sind noch nicht bekannt.

Man erhält sie, wenn man Hydroxyacetamide der Formel (VI),

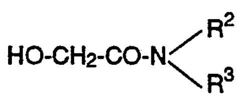
(VI)

in welcher
$R^2$ und $R^3$ die oben angegebenen Bedeutungen haben,
nacheinander in einem "Eintopfverfahren" zunächst mit Schwefelkohlenstoff in Gegenwart einer Base, wie beispielsweise Kaliumhydroxid, dann mit einem ChloressigsäureAlkalisalz, wie beispielsweise Chloressigsäurenatriumsalz, und zuletzt mit einer Säure, wie beispielsweise Salzsäure, gegebenenfalls in Gegenwart eines Verdünnungsmittels, wie beispielsweise Wasser, bei Temperaturen zwischen 0°C und +60°C umsetzt.

Die zur Durchführung des erfindungsgemäßen Verfahren (c) und zur Herstellung der Vorprodukte der Formel (IV) als Ausgangsstoffe benötigten Hydroxyacetamide sind durch die Formel (VI) allgemein definiert. In dieser Formel (VI) stehen $R^2$ und $R^3$ vorzugsweise für diejenigen Reste, die schon bei der Beschreibung der erfindungsgemäßen Stoffe der Formel (I) als bevorzugt für diese Substituenten angegeben wurden.

Die Hydroxyacetamide der Formel (VI) sind bekannt (vgl. z. B. EP-18 497, EP-29 171, DE-OS-30 38 598, DE-OS-31 48 839).

Die weiterhin zur Durchführung der erfindungsgemäßen Verfahren (b) und (c) als Ausgangsstoffe benötigten N-Acylhydrazine sind durch die Formel (V) allgemein definiert. In dieser Formel (V) steht $R^1$ vorzugsweise für diejenigen Reste, die schon bei der Beschreibung der erfindungsgemäßen Stoffe der Formel (I) als bevorzugt für diesen Substituenten genannt wurden.

Die N-Acylhydrazine der Formel (V) sind allgemein bekannte Verbindungen der organischen Chemie oder lassen sich nach prinzipiell bekannten Verfahren in allgemein üblicher Weise durch Acylierung von Hydrazin erhalten (vgl. z. B. C-Ferri, 'Reaktionen der organischen Synthese', Thieme Verlag, Stuttgart 1978, S. 562, 563).

Als Verdünnungsmittel zur Durchführung des erfindungsgemäßen Verfahrens (a) kommen inerte organische Lösungsmittel infrage. Vorzugsweise verwendet man aliphatische oder aromatische, gegebenenfalls halogenierte Kohlenwasserstoffe, wie beispielsweise Benzin, Benzol, Toluol, Xylol, Chlorbenzol, Dichlormethan, Chloroform, Tetrachlorkohlenstoff, Ether, wie Diethylether, Dioxan, Tetrahydrofuran, Ketone, wie Aceton oder Butanon, Nitrile, wie Acetonitril oder Propionitril, Amide, wie Dimethylformamid, Hexamethylphosphorsäuretriamid oder N-Methylpyrrolidon, gegebenenfalls auch Alkohole, wie Methanol, Ethanol, Propanol oder Butanol.

Als Säurebindemittel kommen bei der Durchführung des erfindungsgemäßen Verfahrens (a) alle üblicherweise verwendbaren anorganischen oder organischen Basen infrage. Vorzugsweise verwendet man Alkalialkoholate, wie beispielsweise Natriummethylat oder Natriumethylat oder tertiäre Amine, wie Triethylamin, Pyridin, Dimethylaminopyridin, Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20°C bis +60°C, vorzugsweise zwischen 0°C und +30°C.

Zur Durchführung des erfindungsgemäßen Verfahrens (a) setzt man pro Mol Hydrazin-thiocarbonsäure-O-carbamoylmethylester der Formel (II) im allgemeinen 1 bis 1,5 Mol, vorzugsweise äquimolare Mengen an Acylierungsmittel der Formel (III) und 1 bis 1,5 Mol, vorzugsweise äquimolare Mengen an Säurebindemittel ein.

Zur Aufarbeitung versetzt man den Reaktionsansatz mit Wasser, neutralisiert eventuell im Überschuß vorhandenes Säurebindemittel und isoliert das in Wasser unlösliche Reaktionsprodukt der Formel (I) durch Filtration.

Als Verdünnungsmittel zur Durchführung des erfindungsgemäßen Verfahrens (b) kommen ebenfalls organische Lösungsmittel oder wässrige Systeme infrage. Vorzugsweise verwendet man mit Wasser mischbare Lösungsmittel, wie beispielsweise Methanol, Ethanol oder Acetonitril oder deren Gemische mit Wasser. Besonders bevorzugt ist die Verwendung von Wasser als Verdünnungsmittel.

Als Säurebindemittel kommen bei der Durchführung des erfindungsgemäßen Verfahrens (b) prinzipiell alle üblichen anorganischen und organischen Basen in Betracht. Vorzugsweise verwendet man Alkalimetallcarbonate oder -hydrogencarbonate, wie beispielsweise Natriumhydrogencarbonat oder Kaliumhydrogencarbonat.

Die Reaktionstemperaturen können bei der Durchführung des erfindungsgemäßen Verfahrens (b) ebenfalls in einem größeren Bereich variiert werden. Im allgemeinen arbeitet man bei Temperaturen zwischen -20°C und +50°C, vorzugsweise zwischen 0°C und +30°C.

Zur Durchführung des erfindungsgemäßen Verfahrens (b) setzt man pro Mol O-Carbamoylmethyl-S-carboxymethyl-dithiocarbonat der Formel (IV) im allgemeinen 1 bis 1,3 Mol, vorzugsweise äquimolare Mengen an N-Acylhydrazin der Formel (V) und im allgemeinen 1 bis 1,3 Mol, vorzugsweise äquimolare Mengen an Säurebindemittel ein. Die Aufarbeitung und Isolierung der Reaktionsprodukte der Formel (I) erfolgt, wie bei Verfahren (a) beschrieben.

Als Verdünnungsmittel zur Durchführung des erfindungsgemäßen Verfahrens (c) kommen ebenfalls mit Wasser mischbare inerte organische Verdünnungsmittel oder wässrige Systeme infrage. Vorzugsweise verwendet man reines Wasser.

Als Basen kommen bei der Durchführung des erfindungsgemäßen Verfahrens (c) starke anorganische Basen in Betracht. Vorzugsweise verwendet man Alkalimetallhydroxide, wie beispielsweise Natriumhydroxid oder Kaliumhydroxid.

Die Reaktionstemperaturen können bei der Durchführung des erfindungsgemäßen Verfahrens (c) ebenfalls in einem größeren Bereich variiert werden. Im allgemeinen arbeitet man bei Temperaturen zwischen -20°C und +60°C, vorzugsweise zwischen 0°C und +40°C.

Zur Durchführung des erfindungsgemäßen Verfahrens (c) setzt man pro Mol Hydroxyacetamid der Formel (VI) im allgemeinen 1 bis 1,3 Mol Schwefelkohlenstoff, 1 bis 1,3 Mol Chloressigsäurealkalisalz, wie z. B. Chloressigsäure-Natriumsalz, 1 bis 1,3 Mol N-Acylhydrazin der Formel (V) und 1 bis 1,3 Mol an Base ein, vorzugsweise verwendet man äquimolare Mengen von allen beteiligten Reaktionspartnern. Die Aufarbeitung und Isolierung der Reaktionsprodukte der Formel (I) erfolgt, wie bei Verfahren (a) beschrieben.

Wie schon erwähnt, stellen die erfindungsgemäßen N'-Acylhydrazin-N-thiocarbonsäure-O-carbamoylmethylester der Formel (I) interessante Zwischenprodukte dar.

Sie lassen sich in 1,3,4-Thiadiazol-2-yl-oxyacetamide der allgemeinen Formel (VII),

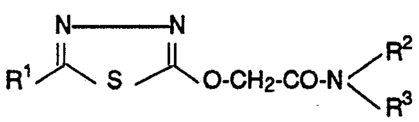

(VII)

in welcher
$R^1$, $R^2$ und $R^3$ die oben angegebenen Bedeutungen haben,
überführen, indem man die Verbindungen der Formel (I) mit einer starken Säure, wie beispielsweise konzentrierter Schwefelsäure, bei Temperaturen zwischen -30°C und +20°C cyclisiert.

Die 1,3,4-Thiadiazol-2-yloxyacetamide der Formel (VII) besitzen herbizide Eigenschaften (vgl. z. B. DE-OS-29 14 003, DE-OS-30 04 326, EP-18 497, DE-OS-29 46 526, EP-29 171, DE-OS-32 18 482).

**Herstellungsbeispiele:**

**Beispiel 1:**

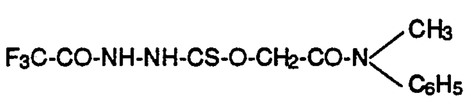

(Verfahren a)

Zu einer Mischung aus 7,1 g (0,05 Mol) Trifluoressigsäureethylester und 40 ml Ethanol tropft man bei 0°C bis 5°C erst eine Lösung von 2,8 g (0,052 Mol) Natriummethanolat in 20 ml Methanol und gibt nach 15 Minuten 11,95 g (0,05 Mol) Hydrazinthiocarbonsäure-O-(N-methyl-N-phenyl-carbamoylmethylester) zu. Man rührt das Gemisch eine Stunde bei 5°C bis 10°C und tropft dann eine Mischung aus 6 ml konzentrierter Salzsäure und 15 ml Wasser zu, Anschließend wird das Lösungsmittel im Vakuum abdestilliert, den Rückstand verrührt man mit kaltem Wasser und saugt das Produkt nach Kristallisation ab.

Man erhält auf diese Weise 14,5 g (87 % der Theorie) N'-Trifluoracetyl-hydrazin-N-thiocarbonsäure-O-(N-methyl-N-phenylcarbamoylmethylester) in Form eines farblosen Pulvers, das bei 107°C unter Zersetzung schmilzt.

**Herstellung der Ausgangsverbindung:**

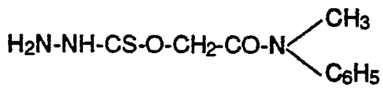

Zu einer Lösung von 9,3 g (0,11 Mol) Natriumhydrogencarbonat in 100 ml Wasser gibt man portionsweise 29,9 g (0,1 Mol) Dithiokohlensäure-S-carboxymethylester-O-(N-methyl-N-phenyl-carbamoylmethylester). Nach einer Stunde tropft man bei 5°C bis 10°C 5 g (0,1 Mol) Hydrazin-Hydrat zu, rührt eine Stunde unter Kühlen nach und saugt das ausgefallene Produkt ab.

Man erhält auf diese Weise 20 g (84 % der Theorie) Hydrazin-thiocarbonsäure-O-(N-methyl-N-phenylcarbamoylmethylester) in Form eines beigen Pulvers mit dem Schmelzpunkt 104°C.

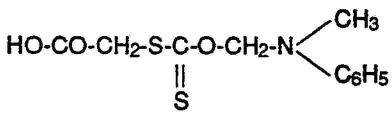

Zu einer Lösung von 5,6 g (0,1 Mol) Kaliumhydroxid in 20 ml Wasser gibt man bei 10°C erst 16,5 g (0,1 Mol) Glykolsäure-N-methylanilid und dann 7,6 g (0,1 Mol) Schwefelkohlenstoff. Han rührt das Gemisch 10 Minuten bei 10°C bis 15°C nach und gibt dann 11,6 g (0,1 Mol) Chloressigsäure-Natriumsalz zu. Die Temperatur steigt dabei bis auf 32°C an. Nach 1,5 Stunden wird mit 40 ml Wasser verdünnt und mit konzentrierter Salzsäure auf pH 2 eingestellt. Das ausgefallene Produkt wird zweimal mit je 100 ml Methylenchlorid extrahiert, die organische Phase wird über Natriumsulfat getrocknet und im Vakuum eingedampft.

Man erhält 27,8 g (93 % der Theorie) Dithiokohlensäure-S-carboxymethylester-O-(N-methyl-N-phenylcarbamoylmethylester) in Form gelber Kristalle mit dem Schmelzpunkt 109°C.

**Beispiel 2:**

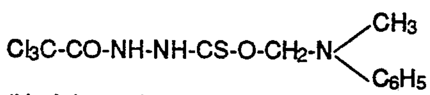

(Verfahren a)

Eine Mischung aus 11,95 g (0,05 Mol) Hydrazinthiocarbonsäure-O-(N-methyl-N-phenyl-carbamoylmethylester) und 25 ml N,N-Dimethylformamid versetzt man bei 0°C bis 5°C erst mit 4 g (0,05 Mol) Pyridin, dann mit 9,1 g (0,05 Mol) Trichloracetylchlorid. Das Gemisch wird eine halbe Stunde bei 0°C bis 5°C nachgerührt und mit 100 ml Eiswasser versetzt. Nach Kristallisation saugt man das ausgefallene Reaktionsprodukt ab.

Man erhält 14,8 g (77 % der Theorie) N'-Trichloracetylhydrazin-N-thiocarbonsäure-O-(N-methyl-N-phenyl-carbamoylmethylester) in Form eines farblosen Pulvers mit dem Schmelzpunkt 104°C (Zersetzung).

**Beispiel 3:**

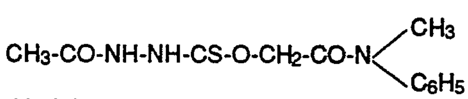

(Verfahren b)

Zu einer Lösung von 8,9 g (0,105 Mol) Natriumhydrogencarbonat in 100 ml Wasser gibt man portionsweise 29,9 g (0,1 Mol) Dithiokohlensäure-S-carboxymethylester-O-(N-methyl-N-phenyl-carbamoylmethylester). Nach einer halben Stunde filtriert man vom Ungelösten und versetzt dann das Filtrat bei 0°C bis 10°C mit 13,5 g (0,1 Mol) einer 55 %-igen wässrigen Acethydrazidlösung. Man rührt das Reaktionsgemisch 6 Stunden bei Raumtemperatur nach und saugt dann das ausgefallene Produkt ab.

Man erhält auf diese Weise 21,7 g (77 % der Theorie) N'-Acetyl-hydrazin-N-thiocarbonsäure-O-(N-methyl-N-phenylcarbamoylmethylester) in Form eines farblosen Pulvers mit dem Schmelzpunkt 115°C.

(Verfahren c)

Zu einer Lösung von 2,8 g (0,05 Mol) Kaliumhydroxid in 10 ml Wasser gibt man bei 20°C erst 8,3 g (0,05 Mol) Glykolsäure-N-methylanilid, dann tropft man bei 10°C bis 20°C 3,8 g (0,05 Mol) Schwefelkohlenstoff zu. Nach 10 Minuten versetzt man das Reaktionsgemisch mit 5,8 g (0,05 Mol) Chloressigsäure-Natriumsalz. Die Temperatur steigt bis auf ca. 30°C an. Nach 2 Stunden tropft man 6,8 g (0,05 Mol) einer 55 %-igen Lösung von Acethydrazid in Wasser zu. Das Gemisch wird 6 Stunden bei Raumtemperatur nachgerührt und dann mit 40 ml kaltem Wasser versetzt; das ausgefallene Produkt wird abgesaugt und mit wenig Eiswasser nachgewaschen.

Auf diese Weise werden 10,8 g (77 % der Theorie) N'-Acetylhydrazin-N-thiocarbonsäure-O-(N-methyl-N-phenyl-carbamoylmethylester) in Form eines farblosen Pulvers mit dem Schmelzpunkt 112°C erhalten.

**Beispiel 4:**

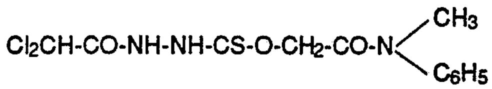

(Verfahren a)

Zu einer Mischung von 119,5 g (0,5 Mol) Hydrazinthiocarbonsäure-O-(N-methyl-N-phenyl-carbamoylmethyl-ester) und 180 ml Acetonitril gibt man bei 0°C bis 5°C 73,8 g (0,5 Mol) Dichloracetylchlorid hinzu. Das Reaktionsgemisch wird 2 Stunden bei 5 - 10°C nachgerührt und dann in eine Lösung von 52 g (0,6 Mol) Natriumbicarbonat in 1700 ml Wasser eingegossen. Das hierbei ausgefallene Reaktionsprodukt wird nach Kristallisation abgesaugt.

Man erhält 157,5 g (90 % der Theorie) N'-Dichloracetylhydrazin-N-thiocarbonsäure-O-(N-methyl-N-phenyl-carbamoylmethyl-ester) in Form eines schwach gelben Pulvers mit dem Schmelzpunkt 85-86°C.

**Beispiel 5:**

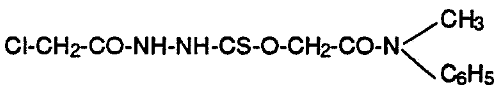

(Verfahren a)

DerN'-Chloracetyl-hydrazin-N-thiocarbonsäure-O-(N-methyl-N-phenyl-carbamoyl-methylester) kann analog zu Beispiel 4 hergestellt werden; man erhält die Verbindung in nicht-kristalliner Form, als Öl.

**Anwendungsbeispiel:**

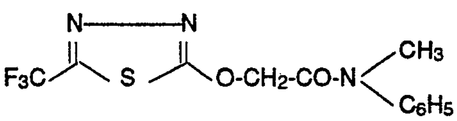

(Herstellung der Folgeprodukte)

16,8 g (0,05 Mol) N'-Trifluoracetyl-hydrazin-N-thiocarbonsäure-O-(N-methyl-N-phenyl-carbamoylmethylester) gibt man bei 0°C bis 5°C zu 50 ml konzentrierter Schwefelsäure. Das Gemisch wird eine Stunde bei 0°C bis 5°C gerührt, auf 500 g Eis gegeben und dann zweimal mit je 100 ml Methylenchlorid ausgeschüttelt. Die organische Phase wird über Natriumsulfat getrocknet und im Vakuum eingedampft.

Man erhält 12,7 g (80 % der Theorie) N-Methyl-N-phenyl-2(5-trifluormethyl-1, 3, 4-thiadiazol-2-yl-oxy)-acetamid vom Schmelzpunkt 54°C.

**Patentansprüche**

1. N'-Acylhydrazin-N-thiocarbonsäure-O-carbamoylmethylester der allgemeinen Formel (I),

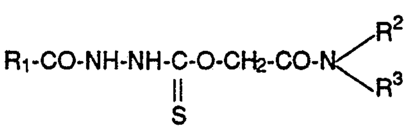 (I)

in welcher

$R^1$ für Alkyl, Alkoxy, Alkylthio, Halogenalkyl, Aralkyl, Aralkoxy, Aralkylthio oder gegebenenfalls substituiertes Aryl steht, und

$R^2$ und $R^3$ unabhängig voneinander für Wasserstoff, Alkyl, Alkenyl, Alkinyl, für jeweils gegebenenfalls substituiertes Cycloalkyl oder Cycloalkenyl, für Halogenalkyl, Alkoxyalkyl und Alkoxy, für Aralkyl und gegebenenfalls substituiertes Aryl stehen, oder

$R^2$ und $R^3$ zusammen mit dem Stickstoffatom, an welches sie gebunden sind, für einen gegebenenfalls substituierten, gesättigten oder ungesättigten Heterocyclus stehen, der weitere Heteroatome enthalten kann.

2. N'-Acylhydrazin-N-thiocarbonsäure-O-carbamoylmethylester der allgemeinen Formel (I) gemäß Anspruch 1, dadurch gekennzeichnet, daß darin

$R^1$ für geradkettiges oder verzweigtes Alkyl, Alkoxy oder Alkylthio mit jeweils 1 bis 6 Kohlenstoffatomen, für Halogenalkyl mit 1 bis 6 Kohlenstoffatomen und 1 bis 13 Halogenatomen, für Aralkyl, Aralkoxy und Aralkylthio mit 6 bis 10 Kohlenstoffatomen im Arylteil und 1 bis 2 Kohlenstoffatomen im Alkylteil sowie für gegebenenfalls einfach oder mehrfach, gleich oder verschieden substituiertes

Aryl mit 6 bis 10 Kohlenstoffatomen steht, wobei als Substituenten in Frage kommen: Alkyl und Alkoxy mit jeweils 1 bis 4 Kohlenstoffatomen, Halogenalkyl mit 1 bis 4 Kohlenstoffatomen und 1 bis 5 Halogenatomen, und daß,

$R^2$ und $R^3$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen, wobei als Substituenten insbesondere Alkylreste mit 1 bis 4 Kohlenstoffatomen in Frage kommen, für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten in Frage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, sowie Nitro, oder daß,

$R^2$ und $R^3$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff und Sauerstoff enthalten kann, wobei als Substituenten in Frage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

3. N'-Acylhydrazin-N-thiocarbonsäure-O-carbamoylmethylester der allgemeinen Formel (I) gemäß Anspruch 1, dadurch gekennzeichnet, daß darin,

$R^1$ für geradkettiges oder verzweigtes Alkyl, Alkoxy oder Alkylthio mit jeweils 1 bis 4 Kohlenstoffatomen, für Halogenalkyl mit 1 bis 4 Kohlenstoffatomen und 1 bis 9 gleichen oder verschiedenen Halogenatomen, für Benzyl oder Benzylthio sowie für gegebenenfalls einfach bis dreifach, gleich oder verschieden substituiertes Phenyl mit den besonders bevorzugten Substituenten: Methyl, Methoxy und Trifluormethyl steht, und daß,

$R^2$ und $R^3$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, gegebenenfalls ein- bis dreifach, gleich oder verschieden durch Methyl oder Ethyl substituiertes Cycloalkyl bzw. Cycloalkenyl mit 5 bis 7 Kohlenstoffatomen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach, gleich oder verschieden substituiertes Phenyl stehen, wobei als Substituenten besonders möglich sind: Methyl, Ethyl, Methoxy, Methylthio, Trifluormethyl, Trifluormethoxy, Trifluormethylthio, Fluor, Chlor, Nitro; oder daß,

$R^2$ und $R^3$ zusammen mit dem Stickstoffatom, an das sie gebunden sind, für einen der folgenden gegebenenfalls ein- bis dreifach, gleich oder verschieden substituierten Heterocyclen:

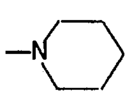 ; 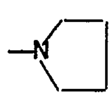 ; 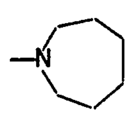 ; 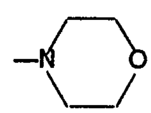 ; 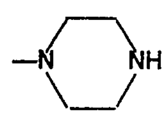 ;

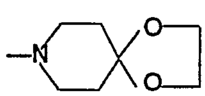 ; 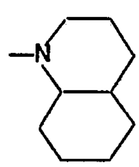 ; 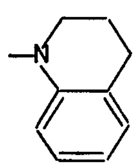 stehen,

wobei als Substituenten möglich sind: Methyl, Ethyl und Phenyl.

4. Verfahren zur Herstellung von N'-Acylhydrazin-N-thiocarbonsäure-O-carbamoylmethylestern der Formel (I) gemäß Anspruch 1, dadurch gekennzeichnet, daß man entweder

(a) Hydrazin-N-thiocarbonsäure-O-carbamoylmethylester der allgemeinen Formel (II),

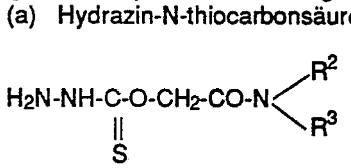 (II)

in welcher

$R^2$ und $R^3$ die oben angegebenen Bedeutungen haben, mit Acylierungsmitteln der Formel (III)

$R^1$-CO-X (III)

in welcher
$R^1$ die oben angegebene Bedeutung hat und
X für Halogen oder Alkoxy steht,
gegebenenfalls in Gegenwart eines Verdünnungsmittels sowie gegebenenfalls in Gegenwart eines Säurebindemittels umsetzt, oder daß man
(b) O-Carbamoylmethyl-S-carboxymethyl-dithiocarbonate der Formel (IV),

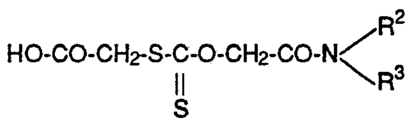
(IV)

in welcher
$R^2$ und $R^3$ die oben angegebene Bedeutungen haben,
mit N-Acylhydrazinen der Formel (V).

$R^1$-CO-NH-$NH_2$ (V)

in welcher
$R^1$ die oben angegebene Bedeutung hat, gegebenenfalls in Gegenwart eines Verdünnungsmittels sowie gegebenenfalls in Gegenwart eines Säurebindemittels umsetzt, oder daß man
(c) Hydroxyacetamide der Formel (VI),

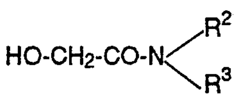
(VI)

in welcher
$R^2$ und $R^3$ die oben angegebene Bedeutungen haben,
nacheinander in einem "Eintopfverfahren" zunächst mit Schwefelskohlenstoff in Gegenwart einer Base, dann mit einem ChloressigsäureAlkalisalz und zuletzt mit einem N-Acylhydrazin der Formel (V),

$R^1$-CO-NH-$NH_2$ (V)

in welcher
$R^1$ die oben angegebene Bedeutung hat,
gegebenenfalls in Gegenwart eines Verdünnungsmittels umsetzt.

## Claims

1. N'-Acylhydrazine-N-thiocarboxylic acid O-carbamoylmethyl esters of the general formula (I)

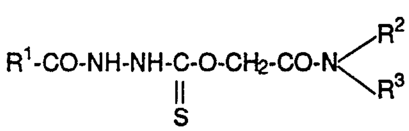
(I)

in which
$R^1$ represents alkyl, alkoxy, alkylthio, halogenoalkyl, aralkyl, aralkoxy, aralkylthio or optionally substituted aryl and,
$R^2$ and $R^3$ independently of one another represent hydrogen, alkyl, alkenyl, alkynyl, in each case optionally substituted cycloalkyl or cycloalkenyl, halogenoalkyl, alkoxyalkyl, alkoxy, aralkyl or optionally substituted aryl, or
$R^2$ and $R^3$ together with the nitrogen atom to which they are bonded, represent an optionally substituted, saturated or unsaturated heterocyclic radical which can contain further heteroatoms.

2. N'-Acylhydrazine-N-thiocarboxylic acid O-carbamoylmethyl esters of the general formula (I) according to Claim 1, characterized in that, in this formula
$R^1$ represents straight-chain or branched alkyl, alkoxy or alkylthio with in each case 1 to 6 carbon atoms, halogenoalkyl with 1 to 6 carbon atoms and 1 to 13 halogen atoms, aralkyl, aralkoxy or aralkylthio with 6 to 10 carbon atoms in the aryl part and 1 or 2 carbon atoms in the alkyl part, or aryl

which has 6 to 10 carbon atoms and is optionally mono- or polysubstituted by identical or different substituents, possible substituents being: alkyl and alkoxy with in each case 1 to 4 carbon atoms and halogenoalkyl with 1 to 4 carbon atoms and 1 to 5 halogen atoms, and in that

$R^2$ and $R^3$ independently of one another represent hydrogen, straight-chain or branched alkyl with 1 to 8 carbon atoms, straight-chain or branched alkenyl or alkynyl with in each case 2 to 8 carbon atoms, cycloalkyl or cycloalkenyl with in each case 3 to 7 carbon atoms and in each case optionally mono- or polysubstituted by identical or different substituents, possible substituents being, in particular, alkyl radicals with 1 to 4 carbon atoms, straight-chain or branched alkoxy or alkoxyalkyl with 1 to 8 carbon atoms, halogenoalkyl with 1 to 8 carbon atoms and 1 to 5 halogen atoms, aralkyl with 6 to 10 carbon atoms in the aryl part and 1 or 2 carbon atoms in the alkyl part, or aryl which has 6 to 10 carbon atoms and is optionally mono- or polysubstituted by identical or different substituents, possible substituents being: halogen, straight-chain or branched alkyl, alkoxy and alkylthio with in each case 1 to 4 carbon atoms, halogenoalkyl, halogenoalkoxy and halogenoalkylthio with in each case 1 or 2 carbon atoms and 1 to 5 halogen atoms, and nitro, or in that

$R^2$ and $R^3$ together with the nitrogen atom to which they are bonded, represent a saturated or unsaturated, 5-membered to 7-membered heterocyclic radical which is optionally mono- or polysubstituted by identical or different substituents and can contain up to 2 further heteroatoms, in particular nitrogen and oxygen, possible substituents being: straight-chain or branched alkyl with 1 to 6 carbon atoms, also in the form of a fused-on ring system, aryl with 6 to 10 carbon atoms, also in the form of a fused-on ring system, or dioxyalkylene with 2 or 3 carbon atoms.

3. N'-Acylhydrazine-N-thiocarboxylic acid O-carbamoylmethyl esters of the general formula (I) according to Claim 1, characterized in that, in this formula,

$R^1$ represents straight-chain or branched alkyl, alkoxy or alkylthio with in each case 1 to 4 carbon atoms, halogenoalkyl with 1 to 4 carbon atoms and 1 to 9 identical or different halogen atoms, benzyl, benzylthio or phenyl which is optionally mono-, di- or trisubstituted by identical or different substituents, particularly preferred substituents being: methyl, methoxy and trifluoromethyl, and in that

$R^2$ and $R^3$ independently of one another represent hydrogen, straight-chain or branched alkyl with 1 to 6 carbon atoms, straight-chain or branched alkenyl or alkynyl with in each case 2 to 6 carbon atoms, cycloalkyl or cycloalkenyl which has 5 to 7 carbon atoms and is optionally mono-, di- or trisubstituted by identical or different substituents from the group comprising methyl and ethyl, halogenoalkyl with 1 to 6 carbon atoms and 1 to 5 halogen atoms, in particular fluorine, bromine and chlorine, benzyl, phenyl which is optionally mono-, di- or trisubstituted by identical or different substituents, particularly possible substituents being: methyl, ethyl, methoxy, methylthio, trifluoromethyl, trifluoromethoxy, trifluoromethylthio, fluorine, chlorine and nitro; or in that

$R^2$ and $R^3$ together with the nitrogen atom to which they are bonded, represent one of the following heterocyclic radicals, which is optionally mono-, di- or trisubstituted by identical or different substituents:

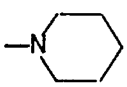 ; 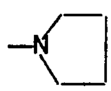 ; 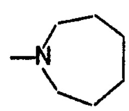 ; 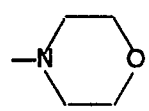 ; 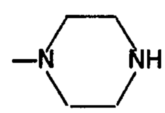 ;

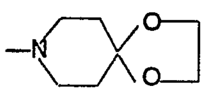 ; 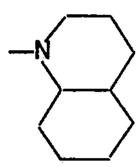 ; 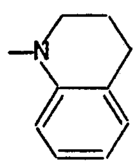

possible substituents being: methyl, ethyl and phenyl.

4. Process for the preparation of N'-Acylhydrazine-N-thiocarboxylic acid O-carbamoylmethyl esters of the formula (I) according to Claim 1, characterized in that either

(a) hydrazine-N-thiocarboxylic acid O-carbamoylmethyl esters of the general formula (II)

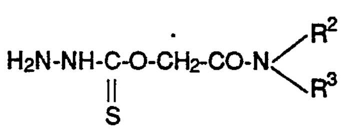 (II)

in which

$R^2$ and $R^3$ have the abovementioned meanings, are reacted with acylating agents of the formula (III)

$R^1$-CO-X (III)

in which

$R^1$ has the abovementioned meaning and,
X represents halogen or alkoxy,
if appropriate in the presence of a diluent and if appropriate in the presence of an acid-binding agent, or:
(b) O-carbamoylmethyl S-carboxymethyl dithiocarbonates of the formula (IV)

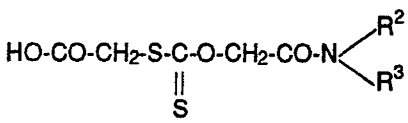
(IV)

in which
$R^2$ and $R^3$ have the abovementioned meanings, are reacted with N-acylhydrazines of the formula (V)

$R^1$-CO-NH-$NH_2$ (V)

in which:
$R^1$ has the abovementioned meaning,
if appropriate in the presence of a diluent and if appropriate in the presence of an acid-binding agent, or
(c) hydroxyacetamides of the formula (VI)

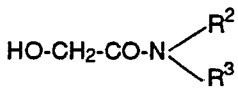
(VI)

in which
$R^2$ and $R^3$ have the abovementioned meanings,
are reacted successively in a "one-pot process" first with carbon disulphide in the presence of a base and then with an alkali metal chloroacetate, and finally with an N-acylhydrazine of the formula (V)

$R^1$-CO-NN-$NH_2$ (V)

in which
$R^1$ has the abovementioned meaning,
if appropriate in the presence of a diluent.

**Revendications**

1. N'-acylhydrazino-N-thiocarboxylates d'O-carbamoylméthyle de formule générale I

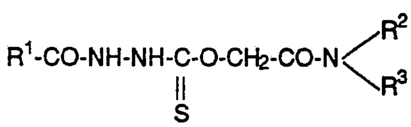
(I)

dans laquelle:
$R^1$ représente un groupe alkyle, alcoxy, alkylthio, halogénoalkyle, aralkyle, aralcoxy, aralkykthio ou un groupe aryle éventuellement substitué, et
$R^2$ et $R^3$ représentent chacun, indépendamment l'un de l'autre, l'hydrogène, un groupe alkyle, alcényle, alcynyle, un groupe cycloalkyle ou cycloalcényle chacun éventuellement substitué, un groupe halogénoalkyle, alcoxyalkyle ou alcoxy, un groupe aralkyle ou un groupe aryle éventuellement substitué, ou bien
$R^2$ et $R^3$ forment ensemble et avec l'atome d'azote auquel ils sont reliés un hétérocycle saturé ou insaturé et éventuellement substitué qui peut contenir d'autres hétéroatomes.

2. N'-acylhydrazino-N-thiocarboxylates d'O-carbamoylméthyle de formule générale I selon la revendication 1, caractérisés en ce que:
$R^1$ représente un groupe alkyle, alcoxy ou alkylthio à chaîne droite ou ramifiée contenant chacun 1 à 6 atomes de carbone, un groupe halogénoalkyle contenant 1 à 6 atomes de carbone et 1 à 13 atomes d'halogènes, un groupe aralkyle, aralcoxy ou aralkylthio contenant 6 à 10 atomes de carbone dans la partie aryle et 1 à 2 atomes de carbone dans la partie alkyle, ou encore un groupe aryle contenant 6 à 10 atomes de carbone et portant éventuellement un ou plusieurs substituants identiques ou différents, les substituants en question étant: des groupes alkyle et alcoxy contenant chacun 1 à 4 atomes de carbone, des groupes halogénoalkyle contenant 1 à 4 atomes de carbone et 1 à 5 atomes d'halogènes, et
$R^2$ et $R^3$ représentent chacun, indépendamment l'un de l'autre, l'hydrogène, un groupe alkyle à chaîne droite

ou ramifiée contenant 1 à 8 atomes de carbone, un groupe alcényle ou alcynyle à chaîne droite ou ramifiée contenant chacun 2 à 8 atomes de carbone, un groupe cycloalkyle ou cycloalcényle contenant chacun 3 à 7 atomes de carbone et portant éventuellement un ou plusieurs substituants identiques ou différents, les substituants étant plus spécialement des groupes alkyle en $C_1$-$C_4$, un groupe alcoxy ou alcoxyalkyle à chaîne droite ou ramifiée en $C_1$-$C_8$, un groupe halogénoalkyle contenant 1 à 8 atomes de carbone et 1 à 5 atomes d'halogènes, un groupe aralkyle contenant 6 à 10 atomes de carbone dans la partie aryle et 1 à 2 atomes de carbone dans la partie alkyle, ou encore un groupe aryle contenant 6 à 10 atomes de carbone et portant éventuellement un ou plusieurs substituants identiques ou différents, les substituants en question étant: des halogènes, des groupes alkyle, alcoxy ou alkylthio à chaîne droite ou ramifiée contenant chacun 1 à 4 atomes de carbone, des groupes halogénoalkyle, halogénoalcoxy et halogénoalkylthio contenant chacun 1 à 2 atomes de carbone et 1 à 5 atomes d'halogènes, ou encore des groupes nitro, ou bien:

$R^2$ et $R^3$ forment ensemble et avec l'atome d'azote auquel ils sont reliés un hétérocycle saturé ou insaturé de 5 à 7 chaînons portant éventuellement un ou plusieurs substituants identiques ou différents et qui peut contenir jusqu'à deux autres hétéroatomes, en particulier d'azote et d'oxygène, les substituants en question étant: des groupes alkyle à chaîne droite ou ramifiée en $C_1$-$C_6$, également sous la forme d'un système cyclique condensé, des groupes aryle en $C_6$-$C_{10}$, également sous la forme d'un système cyclique condensé, ou un groupe dioxyalkylène contenant 2 à 3 atomes de carbone.

3. N'-acylhydrazino-N-thiocarboxylates d'O-carbamoylméthyle de formule générale I selon la revendication 1, caractérisés en ce que:

$R^1$ représente un groupe alkyle, alcoxy ou alkylthio à chaîne droite ou ramifiée contenant chacun 1 à 4 atomes de carbone, halogénoalkyle contenant 1 à 4 atomes de carbone et 1 à 9 atomes d'halogènes identiques ou différents, benzyle ou benzylthio ou encore phényle portant éventuellement 1 à 3 substituants identiques ou différents, les substituants les plus appréciés étant les groupes méthyle, méthoxy et trifluorométhyle, et

$R^2$ et $R^3$ représentent chacun, indépendamment l'un de l'autre, l'hydrogène, un groupe alkyle à chaîne droite ou ramifiée en contenant un groupe alcényle ou alcynyle à chaîne droite ou ramifiée contenant chacun 2 à 6 atomes de carbone, un groupe cycloalkyle ou cycloalcényle en $C_5$-$C_7$ portant éventuellement 1 à 3 substituants identiques ou différents choisis parmi les groupes méthyle ou éthyle, un groupe halogénoalkyle contenant 1 à 6 atomes de carbone et 1 à 5 atomes d'halogènes, en particulier de fluor, de brome et de chlore, un groupe benzyle ou un groupe phényle portant éventuellement 1 à 3 substituants identiques ou différents, les substituants consistant particulièrement en: des groupes méthyle, éthyle, méthoxy, méthylthio, trifluorométhyle, trifluorométhoxy, trifluorométhylthio, le fluor, le chlore, des groupes nitro; ou bien

$R^2$ et $R^3$ forment ensemble et avec l'atome d'azote auquel ils sont reliés l'un des hétérocycles suivants:

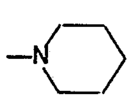 ; 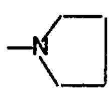 ; 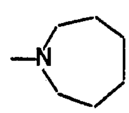 ; 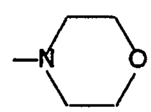 ; 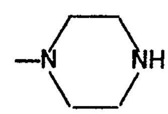 ;

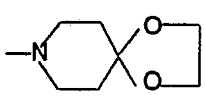 ; 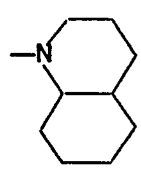 ; 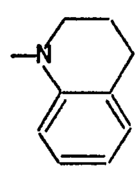

portant éventuellement 1 à 3 substituants identiques ou différents, les substituants possibles étant des groupes méthyle, éthyle et phényle.

4. Procédé de préparation des N'-acylhydrazino-N-thiocarboxylates d'O-carbamoylméthyle de formule I selon la revendication 1, caractérisé en ce que:

a) on fait réagir des hydrazino-N-thiocarboxylates d'O-carbamoylméthyle de formule générale II

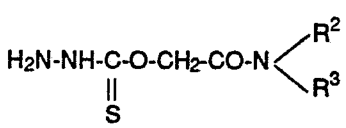 (II)

dans laquelle

$R^2$ et $R^3$ ont les significations indiquées ci-dessus, avec des agents acylants de formule III

$R^1$-CO-X (III)

dans laquelle

$R^1$ a les significations indiquées ci-dessus, et
X représente un halogène ou un groupe alcoxy,
éventuellement en présence d'un diluant et éventuellement en présence d'un capteur d'acide, ou bien
b) on fait réagir des dithiocarbonates d'O-carbamoylméthyle et de S-carboxyméthyle de formule IV

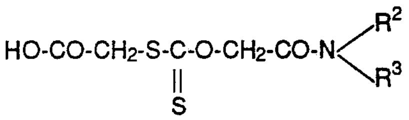 (IV)

dans laquelle
$R^2$ et $R^3$ ont les significations indiquées ci-dessus, avec des N-acylhydrazines de formule V

$R^1$-CO-NH-$NH_2$ (V)

dans laquelle
$R^1$ a les significations indiquées ci-dessus,
éventuellement en présence d'un diluant et éventuellement en présence d'un capteur d'acide, ou bien
c) on fait réagir des hydroxyacétamides de formule VI

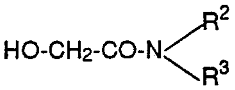 (VI)

dans laquelle
$R^2$ et $R^3$ ont les significations indiquées ci-dessus,
successivement, dans une "opération en un seul récipient" d'abord avec le sulfure de carbone en présence d'une base, puis avec un chloracétate de métal alcalin et finalement avec une N-acylhydrazine de formule V

$R^1$-CO-NH-$NH_2$ (V)

dans laquelle
$R^1$ a les significaticns indiquées ci-dessus,
éventuellement en présence d'un diluant.